(19) **Europäisches Patentamt / European Patent Office / Office européen des brevets**

(11) **EP 4 518 751 B1**

(12) **EUROPEAN PATENT SPECIFICATION**

(45) Date of publication and mention of the grant of the patent:
**14.01.2026  Bulletin 2026/03**

(21) Application number: **23725296.0**

(22) Date of filing: **26.04.2023**

(51) International Patent Classification (IPC):
**A61B 5/11** (2006.01)    **G01S 13/56** (2006.01)
**G01S 13/58** (2006.01)    **G01S 7/00** (2006.01)
**G01S 7/288** (2006.01)    **A61B 5/00** (2006.01)
**G01S 13/526** (2006.01)

(52) Cooperative Patent Classification (CPC):
**A61B 5/1123; A61B 5/1126; G01S 7/006;
G01S 7/2883; G01S 13/56; G01S 13/582;**
A61B 5/1114; A61B 5/7257; A61B 5/7267;
G01S 13/526; G01S 2205/09

(86) International application number:
**PCT/IB2023/054279**

(87) International publication number:
**WO 2023/214252 (09.11.2023 Gazette 2023/45)**

(54) **METHOD AND SYSTEM FOR THE SPARSE RECONSTRUCTION OF THE MICRO-DOPPLER SPECTRUM IN JOINT COMMUNICATION AND SENSING APPLICATIONS**

VERFAHREN UND SYSTEM ZUR SPÄRLICHEN REKONSTRUKTION DES MIKRO-DOPPLER-SPEKTRUMS BEI GEMEINSAMEN KOMMUNIKATIONS- UND ERFASSUNGSANWENDUNGEN

PROCÉDÉ ET SYSTÈME DE RECONSTRUCTION ÉPARSE DU SPECTRE MICRO-DOPPLER DANS UNE COMMUNICATION CONJOINTE ET UNE APPLICATION DE DÉTECTION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **03.05.2022  IT 202200008906**

(43) Date of publication of application:
**12.03.2025  Bulletin 2025/11**

(73) Proprietors:
• **Universita' Degli Studi Di Padova**
  **35122 Padova (IT)**
• **Fundación Imdea Networks**
  **28918 Leganés - Madrid (ES)**

(72) Inventors:
• **ROSSI, Michele**
  **44124 Ferrara (FE) (IT)**
• **PEGORARO, Jacopo**
  **30033 Noale (VE) (IT)**
• **LACRUZ JUCHT, Jesus Omar**
  **28913 Leganés Madrid (ES)**
• **WIDMER, Joerg**
  **28023 Madrid Madrid (ES)**

(74) Representative: **Soranzo, Benedetta et al**
  **Società Italiana Brevetti S.p.A.**
  **Stradone San Fermo, 21**
  **37121 Verona (IT)**

(56) References cited:
• **JACOPO PEGORARO ET AL: "RAPID: Retrofitting IEEE 802.11ay Access Points for Indoor Human Detection and Sensing",** ARXIV.ORG, CORNELL UNIVERSITY LIBRARY, 201 OLIN LIBRARY CORNELL UNIVERSITY ITHACA, NY 14853, 30 March 2022 (2022-03-30), XP091171084

- MENEGHELLO FRANCESCA ET AL: "Environment and Person Independent Activity Recognition with a Commodity IEEE 802.11ac Access Point", 17 March 2021 (2021-03-17), pages 1 - 13, XP093007751, Retrieved from the Internet <URL:https://arxiv.org/pdf/2103.09924v1.pdf> [retrieved on 20221213]
- TAN BO ET AL: "Using Wi-Fi Signal as Sensing Medium: Passive Radar, Channel State Information and Followups A Brief Rewind", 9 January 2022 (2022-01-09), pages 1 - 4, XP093008093, Retrieved from the Internet <URL:https://arxiv.org/ftp/arxiv/papers/2201/2201.03005.pdf> [retrieved on 20221214]
- PEGORARO JACOPO ET AL: "SPARCS: A Sparse Recovery Approach for Integrated Communication and Human Sensing in mmWave Systems", 2022 21ST ACM/IEEE INTERNATIONAL CONFERENCE ON INFORMATION PROCESSING IN SENSOR NETWORKS (IPSN), IEEE, 4 May 2022 (2022-05-04), pages 79 - 91, XP034151762, DOI: 10.1109/IPSN54338.2022.00014

## Description

The present invention refers to a method and system for joint communication and reconstruction of the micro-doppler time-frequency spectrum from sparse channel measurements.

## Background

A well established method to detect and classify human movements using radiofrequency transmitting and receiving devices is the time-frequency analysis of the small-scale Doppler effect (termed micro-Doppler) of the different body parts, which requires a regularly spaced and dense sampling of the Channel Impulse Response (CIR). This is currently done in the literature either using special-purpose radar sensors, or interrupting communications to transmit dedicated sensing waveforms, entailing high overhead and channel utilization.

There is a growing interest in human tracking and person identification using devices, where the high carrier frequency and large available bandwidth allow for accurate Doppler estimation and precise localization and tracking. To fully exploit these properties, a large body of work has focused on dedicated radars, that adopt specifically designed frequency modulated transmissions to extract the micro-Doppler ($\mu$D) the effect caused by human motion (a so-called $\mu$D signature).

At the same time, given the increasing number of network technologies such as 3GPP LTE and 5G-NR and IEEE 802.11, solutions are highly appealing. They effectively repurpose communication devices by endowing them with additional environment sensing capabilities, thus avoiding the cost of installing dedicated radar hardware. This recent trend has led to the identification of sensing as a key feature of next generation 6G mobile networks and the creation of the IEEE 802.11bf standardization group, aimed at enabling sensing features in Wireless Local Area Networks (WLANs). However, system designs are still very limited, focusing on joint communication and sensing waveform design, which would require significant modifications to existing communication protocols and a reduction in the achievable communication data rates. Other approaches need to alternate communication and sensing phases according to a time-division scheme, where regularly spaced, radar-like transmissions are performed during dedicated sensing periods. This is needed to perceive the fine-grained effect of human motion, for which dense and regular sampling of the Channel Impulse Response (CIR) is required, causing significant overhead and channel occupation.

The high sensitivity of microwaves to micro-Doppler shifts, together with Deep Learning (DL) methods for spectrogram analysis and classification, have been widely exploited to enable applications such as activity recognition, person identification and bio-mechanical gait analysis.

The typical approach in the prior art is to employ dedicated radar devices working in the millimeter-wave (mmWave) frequency band to transmit sequences of large bandwidth signals (of 2 to 4 GHz), with a rate dictated by the desired sensing resolution. Thus, mmWave radar sensors have two main drawbacks:

(i) they are specifically tailored to sensing and cannot perform communication. Moreover, their deployment cost is relatively high as one single sensor can reliably cover a range of at most 8 - 10 m due to the radial distortion and occlusion problems. For this reason, ad-hoc radar sensor networks would need to be deployed in practical scenarios. The method according to the invention, in contrast, fully exploits existing communication systems with no modifications to the CIR estimation process or packet structures;

(ii) the fixed chirp transmission interval, which is related to the $\mu$D resolution, requires regular transmissions with continuous channel occupation. Some works have explored the possibility of randomly subsampling the chirp transmission intervals using compressive sensing to either save computational resources or reduce the effect of unwanted interference. However, these works are based on a radar framework, where the transmission instants can be freely chosen and optimized.

In Jacopo Pegoraro et al.: "RAPID: Retrofitting IEE 802.11ay Access Points for Indoor Human Detection and Sensing", ARXIV.ORG, CORNELL UNIVERSITY LIBRARY, 201 OLIN LIBRARY CORNELL UNIVERSITY ITHACA, NY 14853, 30 March 2022, XP091171084, a method to track people using the channel impulse response obtained with IEEE 802.11ay-compliant packet transmission is disclosed. It also presents a way to extract micro-Doppler signatures of human movement using the IEEE 802.11ay channel impulse response, and demonstrates that it can be used to perform human activity recognition. However, to do so, it requires that 802.11ay packets are transmitted regularly with a fixed inter-packet time. If this condition is not met, the quality of the extracted micro-Doppler degrades, and the activity recognition accuracy drops. This makes the method hard to apply in practice, as IEEE 802.11ay (WiFi) communication traffic is highly irregular and sparse.

The present invention, instead, reuses the given underlying communication traffic as much as possible and only injects small additional sensing units when necessary.

Research interest towards sensing with WiFi devices has mostly focused on the IEEE 802.11n/ac/ad/ay standards. The prior art targets various applications, such as person tracking and gesture recognition, exploiting CIR estimation to detect humans in the environment. However, they require dedicated and regular sensing signal transmissions in order to function properly, entailing a significant overhead and channel utilization for sensing.

The present invention significantly improves over the above mentioned studies in that it enables the reuse of randomly distributed communication packets via sparse recovery, whenever possible. This is of key importance to integrate sensing capabilities in communication devices while maintaining low overhead and complexity.

A number of technical works address ISAC systems in next generation 5G/6G cellular networks and WLANs. Many of those target the joint communication and sensing waveform design and are mostly oriented to automotive applications to measure distance and velocity of nearby vehicles. In contrast, few works focus on human sensing, which is the aim of the present work. All of the above approaches alternate communication and sensing phases according to a time-division scheme, causing significant overhead and channel occupation.

The present invention, instead, provides a full ISAC scheme, as it passively exploits communication traffic while dynamically injecting sensing units to cover silent periods. As a result, the present invention significantly reduces sensing overhead while at the same time improving the sensing accuracy.

## Summary of the invention

According to the present invention an integrated human sensing and communication solution is provided. The present invention provides a method that reconstructs high quality signatures of human movement from irregular and sparse samples, such as the ones obtained during communication traffic patterns. To accomplish this, the micro-Doppler extraction has been formulated as a sparse recovery problem, which is critical to enable a smooth integration between communication and sensing. Moreover, if needed, the proposed system and method can seamlessly inject short estimation fields into the channel whenever communication traffic is absent or insufficient for the micro-Doppler extraction. The present invention effectively leverages the intrinsic sparsity of the channel, thus drastically reducing the sensing overhead with respect to available approaches. The present invention has been implemented and tested on an IEEE 802.11ay platform working in the 60 GHz band, collecting standard-compliant traces matching the traffic patterns of real WiFi access points (AP). The results show that the micro-Doppler signatures obtained by the present invention enable a typical downstream application such as human activity recognition with more than seven times lower overhead with respect to existing methods, while achieving better recognition performance.

According to the present invention the problem of enabling sensing capabilities in realistic communication systems is addressed, by reusing existing communication traffic for sensing as much as possible and thus introducing only a minimal amount of additional overhead. To this aim, the present invention is proposed, the first system that reconstructs human signatures from irregular and sparse samples obtained from realistic traffic patterns. The main insight of the present invention is to leverage the intrinsic sparsity of the reflections in the channel to pose the reconstruction as a sparse recovery problem. This allows obtaining highly accurate signatures from only a small, randomly distributed fraction of the samples that are currently needed by existing methods. To do so, the present invention first performs resampling to construct a regular grid of samples with missing values due to the irregularity of the sampling process in time. Next, a sparse reconstruction method is used to obtain the spectrum, decoupling different propagation paths to leverage their sparsity property. Lastly, whenever communication traffic is absent or insufficient for the extraction, the present invention supports a dynamic injection of very short estimation fields into the (idle) channel. Given its sparse recovery capabilities, only a small number of additional sensing units are needed to retrieve the $\mu$D, thus entailing a negligible overhead to the communication rate.

The solution according to the present invention inserts in this context, the invention proposing to provide a system and a method for joint communication and reconstruction of the micro-doppler time-frequency spectrum from sparse channel measurements.

The object of the present invention is then to solve the problems left unsolved by the known art, by providing a computer implemented method as defined in claim 1.

The present invention further relates to a system as defined in the independent claim 14.

Additional features of the present invention are defined in the corresponding depending claims.

The present invention is compatible with any communication system, including those supporting transmit beamforming for directional communication and channel estimation. This is the case, for example, for IEEE 802.11ay at 60GHz, which provides in-packet estimation for beam tracking purposes, and for 3GPP 5G-NR, where base stations can send frequent downlink packet fields to estimate the channel using different Beam Patterns (BP).

To evaluate its performance, the invention has been implemented on a 60 GHz IEEE 802.11ay experimentation platform. It has been tested on sparse and irregular samples derived from standard-compliant traces, both for synthetic traffic and traffic patterns obtained from datasets of operational real-world WiFi deployments. To assess the quality of the reconstructed signatures, they have been used as input for a typical downstream task such as, which classifies human movement detected by the captured into different possible activities. The main contributions of the present invention are summarized next.

The present invention is proposed, a method for systems that can reconstruct high-quality signatures of human movement from irregular and sparse estimation samples. The present invention can reuse training fields appended to communication packets as sensing units, and inject additional sensing units if necessary, adapting to the underlying communication traffic and minimizing the sensing overhead.

An original formulation of the extraction in communication systems as a sparse recovery problem is provided, leveraging the intrinsic high distance resolution and sparsity properties of the channel. As a side effect, this also improves the quality of the resulting spectrograms, making them more robust to noise and interference.

An algorithm to perform the injection of additional sensing units when communication traffic is insufficient has been designed and validated. The process is dynamic, requires no knowledge about future packet transmissions, and incurs minimal overall overhead.

The present invention has been evaluated by implementing it on an IEEE 802.11ay-compliant 60 GHz platform and testing it on measurements collected with realistic WiFi traffic patterns. For the common task, the signatures reconstructed by the invention achieve better F1 scores in human activity recognition than existing methods, while reducing sensing overhead by a factor of seven. The F1-score is defined as TP/[TP + 0.5(FP + FN)], where TP, FP and FN are the predicted true positives, false positives and false negatives, respectively.

Other advantages, together with the features and use modes of the present invention, will result evident from the following detailed description of preferred embodiments thereof, shown by way of example and not for limitative purposes.

## Brief description of the figures

Hereinafter in this description, reference will be made to the drawings shown in the enclosed figures, wherein:

- Figure 1 is a comparison between the traditional CIR-based human sensing and the present invention.

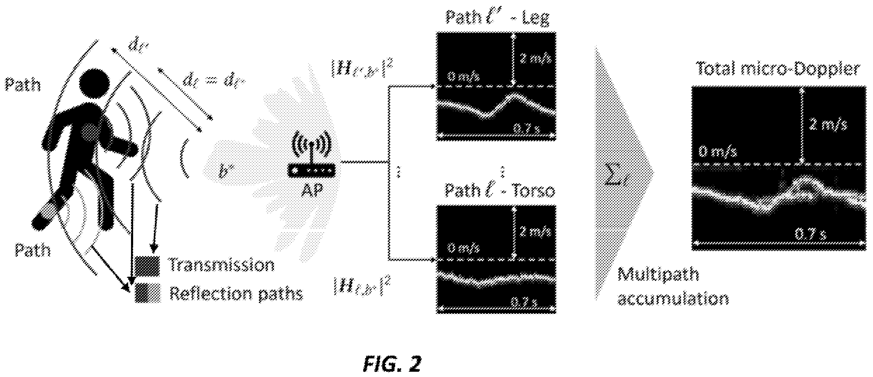

- Figure 2 is a visual representation of the $\mu$D spectrum computed using the present invention on two different CIR paths, one containing the reflection from a person's torso, the other capturing the $\mu$D signature of the leg. The total $\mu$D is obtained summing together these contributions.
- Figure 3 shows an example injection procedure with $M_s = 8$, $W = 16$. Four sensing units are scheduled after P1 and P2. Then, as three reflected communication packets are received, they are reused and the first three scheduled sensing units are not transmitted. The fourth sensing unit is instead injected in the last slot.
- Figure 4 shows an exemplary block diagram of a system according to the invention.
- Figure 5 shows walking $\mu$D spectrograms and RMSE for different levels of sparsity, obtained by uniformly removing samples for each window.
- Figure 6 shows exemplary traffic patterns from the pdx/vwave dataset.
- Figure 7 shows per-class F1 scores obtained for different values of $M_s$ and RAPID on the tested dataset.
- Figure 8A shows overhead of the invention for different values of $M_s$ in the three traces of the pdx/vwave dataset.
- Figure 8B shows overhead vs. average HAR F1 score for different values of $M_s$.
- Figure 9A shows per-class F1 scores aggregating a different number of paths $Q$.
- Figure 9B shows per-class F1 scores changing $\Omega$, the IHT sparsity parameter.

## Technical background

In the following, a brief description of the CIR model for communication systems used for sensing is provided. Then a baseline approach is described that allows to track the movement of people in the environment and extract their $\mu$D signatures using regularly sampled CIR information. This forms the basis of the present invention, which eliminates the requirement of fixed Inter-Frame Spacing (IFS) and enables ultra low-overhead ISAC.

### *Sensing in communication systems*

Capturing the movement features of humans in the environment requires an analysis of the reflections of the transmitted signal from their bodies, which is usually carried out applying signal processing techniques to the CIR. In the case of standard communication systems, for example, network devices implementing the IEEE 802.11 standard at 2.4 or 5 GHz, communication is typically performed omnidirectionally. For this reason, and due to the intrinsic lower distance resolution of such frequencies, super-resolution techniques would be needed to accurately localize and sense the subjects. In the case of mmWave systems instead, due to the high path loss occurring at mmWave frequencies, directional communication is employed by means of transmitter and receiver beamforming, typically by means of phased antenna arrays. The transmitter and the receiver use suitable BP configurations of their antenna arrays to maximize the signal strength. To successfully sense with a mmWave system, at least one of the BPs has to illuminate the subjects, as only in this case the reflected signal carries detectable information about the movement signature. To this end, in the testing of the present invention a setup is considered where an AP transmits packets and is able to collect the reflections of its own signal, after being reflected by objects (including humans). This reflection is preferably collected by the receive array of the AP itself using a quasi-omnidirectional BP. This requires full-duplex capabilities, as is common in ISAC scenarios, which in the simplest form can be achieved with a Multiple Input Multiple Output (MIMO) system in a mixed configuration with one RF chain as transmitter and another as receiver. The full-duplex configuration is not critical for the functioning of the proposed method and only constitutes a preferred embodiment used in the evaluation of the invention. The sensing-oriented CIR estimation fields, which are denoted by sensing units, can either be transmitted independently (injected) or piggybacked by appending them as a trailer to the Physical Layer (PHY) communication packets. mmWave standards implement beam training mechanisms that help to establish a communication link by testing different BP combinations and then selecting the best one. Such functionality is supported by all mmWave standards. For example, 5G-NR, use Synchronization Signal Block (SSB) and CSI-RS for beam management, while WLAN systems adopting the IEEE 802.11ad/ay standards use channel estimation and training fields (CEF and TRN, respectively) to obtain accurate CIR information. The framework to extract sensing information from CIR measurements can be applied regardless of the specifics of the standards.

*CIR model*

Channel measurements in communication systems contain information about the environment. Depending on the communication system, sensing could be performed using, e.g., the 5G-NR and IEEE 802.11n/ac/ax Orthogonal Frequency Division Multiplexing (OFDM) Channel State Information (CSI), which contains the channel gains for each OFDM subcarrier, or the IEEE 802.11ad/ay Single Carrier (SC) CIR.

Both communication schemes are suitable for human sensing: (i) in OFDM systems, the base stations can send frequent downlink packets to estimate the channel, possibly using different BPs if transmit beamforming is enabled, while (ii) in SC systems, such as IEEE 802.11ad/ay, in-packet beam tracking can be used, so that specific fields called training fields (TRN), each using a different BP, can be appended to communication packets. In the following, the focus will be on SC CIR, and it will be shown how to extract the $\mu$D effect of human movement. However, previous works have demonstrated that similar processing can be performed with OFDM CSI, and the present invention is general enough to be applied in both cases. In addition, the CIR model presented in the following assumes that the transmitter device can implement transmitter beamforming to direct the signal energy along preferred directions. This is coherent with the preferred implementation of the system but the proposed method is general enough to be applied to systems that do not implement transmitter beamforming.

The CIR contains the complex channel gains for each path $\ell = 0, ... , L$ - 1 along which the signal travels. Each path is associated to a specific distance from the AP, according to the relation $d_\ell = c\tau_\ell/2$, with $\tau_\ell$ being the delay of the reflection from path $\ell$ and $c$ the speed of light. The ranging resolution of the system, i.e., its capability to resolve the distance of the reflectors causing different signal paths, is given by $\Delta d = c/2B$, termed range bin, where $B$ is the bandwidth of the transmitted signal. Moreover, the CIR depends on the specific BP used during the transmission, denoted by $b = 0, ... , N_{BP}$ - 1.

For carrier frequency $f_o$, the CIR along $\ell$, using BP $b$ at time $t$ is

$$h_{\ell,b}(t) = \sum_{p=1}^{P_\ell(t)} a_{\ell,b}^p(t)\exp\left\{-j2\pi\frac{2f_o}{c}\left[d_\ell + \int_0^t v_\ell^p(x)dx\right]\right\}. \qquad (1)$$

In Eq. (1), $P_\ell(t)$ is the number of reflectors that are located within $\Delta d$ from $d_\ell$, whose contributions are overlapping in path $\ell$, while $v_\ell^p$ is the radial velocity (by convention, $v_\ell^p$ has a positive sign when the reflector moves away from the AP) of the $p$-th reflector. The quantity $a_{\ell,b}^p(t)$ contains the complex gain due to the joint effect of the transmitter BP, the reflectivity of the object and the signal attenuation.

*micro-Doppler extraction*

The extraction of the $\mu$D spectrum from multiple, concurrently moving subjects requires tracking the position of each person in the physical space, in order to separate their individual contributions to the CIR. Then, a spectral analysis over different CIR samples yields the desired $\mu$D signature.

*People tracking.*

In a possible embodiment of the present invention, a people tracking phase can precede the micro-Doppler extraction, in order to perform the extraction separately for each subject to be sensed. People tracking is performed by extracting measurements of each person's distance and angular position with respect to the AP across time. This process consists of (i) removing the background contribution to the CIR by subtracting the average CIR across a suitable time interval, (ii) selecting the locally strongest reflection paths in the CIR (peaks) and obtaining the corresponding distance $d\ell$, (iii) computing the Angle of Arrival (AoA), $\theta$, of the reflection from the correlation between the different BPs gains and the strengths of the CIRs across the whole angular Field-of-View (FoV). The approach in (iii) requires the BP shapes to be estimated in advance, and is based on the intuition that different BP illuminate different possible reflectors in the environment, depending on their position, so that one can expect to receive the reflected signal from a certain subject only when a BP pointing in his/her direction is used. The resulting set of distances and angles represent candidate positions of humans in the environment. A multi-target tracking method such as a Joint Probabilistic Data Association Filter (JPDAF) allows smoothing the trajectories of the subjects rejecting noise and clutter.

The tracking phase provides an estimate of the position of each subject, at every time instant $t$, which is denoted by $[\hat{d}(t), \hat{\theta}(t)]$ using the superscript to differentiate between the estimate and the true position. Due to step (ii) above, which selects the local peaks in the received power, typically only the reflections from the torso can be tracked when a person moves in the environment. This is because the head and the limbs cause much weaker reflections, whose contributions can only be detected in the $\mu$D spectrum. Nevertheless, tracking the spatial location of the torso is crucial for the $\mu$D extraction as it allows separating the contributions of the multiple subjects in the environment. Once $\hat{d}(t)$ and $\hat{\theta}(t)$ are determined, they are used to select the path $\ell^*$ and the BP $b^*$ that correspond to the distance and angular position of the subject, respectively.

Then, the CIR waveform that contains the $\mu$D effect of the person's movement is $h_{\ell^*,b^*}(t)$. Besides enabling the separation of multiple subjects, this operation makes mmWave sensing systems much more robust to changes in the environment than sub-6 GHz devices. While the latter are heavily affected by second-order reflections on walls and objects, mmWave sensing mostly relies on the line-of-sight path between the person and the transmitter/receiver, with little contribution from the external environment.

*micro-Doppler spectrum.*

Human movement causes a small scale Doppler effect on the reflected signal due to the different body parts, which possess different velocities and follow different trajectories. This is referred to as $\mu$D effect and causes a measurable frequency modulation on the reflection. High frequency signals such as mmWave communications are particularly affected by the $\mu$D modulation due to their small wavelengths. Various techniques from time-frequency analysis can be applied to analyze the $\mu$D, obtaining spectrograms showing the time evolution of the signal energy contained in the different frequency bands of interest. The most popular and computationally efficient of such methods is the Short Time Fourier Transform (STFT) of $h_{\ell,b}(t)$, which consists in applying a windowed Fourier Transform (FT) on partially overlapping portions of the CIR. STFT processing needs to be applied on a window of W subsequent estimates of the CIR, computed with a fixed sampling period of $T_c$ seconds, provided that the time spanned by the window is short enough to consider the movement velocity of the reflectors constant for its whole duration. Note that this operation allows to detect and separate the velocities of the $P_\ell(t)$ reflectors, whose contributions are overlapping in path $\ell$ when considering a single estimate of the CIR. The choice of $T_c$ impacts the frequency resolution of the STFT, $\Delta f^d = 1/(WT_c)$, and its maximum measurable frequency, $f_{max}^d = 1/(2T_c)$. Using the relationship between the Doppler frequency and the corresponding velocity, one can obtain the velocity resolution and the maximum observable velocity as $\Delta v = c/(2f_oWT_c)$ and $v_{max} = c/(4f_oT_c)$. To fully capture the range of velocities of interest for human movement, the typical approach is to select $T_c$ such that $v_{max}$ is sufficiently high that is covers the velocities that can occur in the human activities of interest, which may vary depending on the application.

Previous work assumes that the constraint of a fixed $T_c$ is met, which does not hold in realistic communication scenarios, where the packet transmissions are scheduled according to the needs of the communication protocols rather than sensing accuracy. Traffic patterns are typically bursty and irregular and thus cannot be used by existing methods for human sensing. Instead, dedicated time slots need to be reserved for the transmission of sensing units, which is incompatible with the random access CSMA/CA MAC commonly used in IEEE 802.11. Conversely, the present invention is the first approach that does not require any specific pattern in the transmission of the sensing units, thus enabling a true ISAC where communication packets are exploited for sensing whenever possible, and only a minimal additional overhead is introduced when necessary.

**Detailed description of preferred embodiments**

The present invention will be described hereinafter by making reference to the above-mentioned figures.

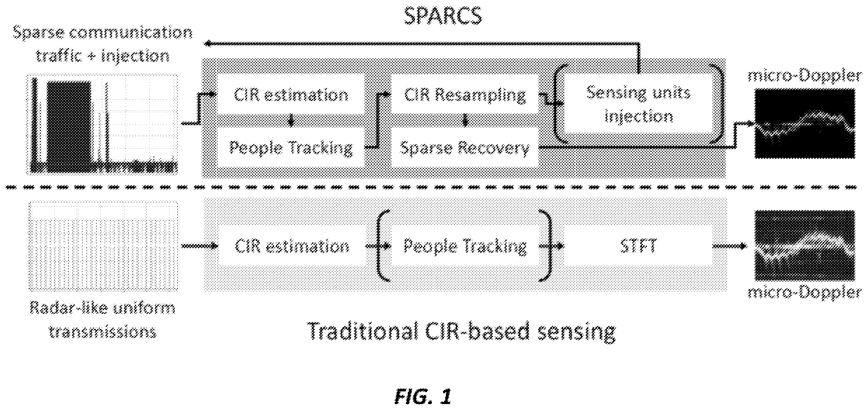

The algorithm to recover the $\mu$D spectrum from irregular and sparse CIR sampling patterns according to the present invention is now presented. The processing steps of the method according to the present invention, compared to traditional CIR-based sensing methods are shown in Fig. 1.

In general terms, a method according to the present invention allows joint communication and reconstruction of the micro-Doppler time-frequency spectrum from sparse channel measurements, wherein wireless communication signals, in the form of packets including channel estimation fields are transmitted through a multi-path channel, and the reflection or refraction of the transmitted signal are received.

Such method essentially comprises the following steps:

i) estimating the channel impulse response (CIR) of the multi-path channel;
ii) resampling the available channel impulse response (CIR) estimates to an incomplete regular grid of CIR measurements using a resampling technique;
iii) performing sparse reconstruction of the Fourier transform of the incomplete regular grid in the time domain.

In particular, after CIR estimation and, eventually, people tracking, for which, for example, the standard JPDAF technique can be adopted, a resampling technique to approximate the irregularly spaced CIR values with a regular grid whose sampling interval is chosen according to the desired $\mu$D resolution. Due to irregularity of the original sampling process, the approximated regular sequence may be incomplete, containing missing values that need to be filled in the subsequent processing steps.

The recovery of the $\mu$D spectrum from the resampled incomplete regular grid of CIR measurements is formulated as a sparse recovery problem. For this, the intrinsic sparsity of the propagation channel is leveraged, which leads to a small number of signal reflections from the human body, each carrying information about a different body part.

According to preferred embodiments, the sparse recovery problem is preferably solved with an algorithm using the Iterative Hard Thresholding (IHT) method for each signal propagation path (CIR path), and aggregate the results to obtain the final $\mu$D spectrum. The aggregation may be, for example performed by summing the micro-Doppler spectra obtained from the Fourier transforms.

According to possible embodiments of the invention, when communication traffic is absent or too scarce to obtain an accurate reconstruction, according to the invention short sensing units can be injected into the (idle) channel to overcome the problem. Thanks to the sparse reconstruction of point (2), the amount of units that need to be injected is minimal and can be tuned to trade off between overhead and sensing accuracy.

In particular the resampling is performed on a slotted sliding window with predetermined slot duration (T) and window length slots (W), and wherein the steps i), ii) and iii) are repeated for each subsequent window.

Then, the following steps are performed between said estimating and resampling steps:

a) setting a threshold number of CIR measurements-per-window, needed to reach the desired micro-Doppler reconstruction quality;
b) transmitting a number of additional CIR estimation fields to meet the threshold number of measurements, if the communication traffic is not sufficient.

According possible embodiments of the invention, the time slot duration $T$ is selected as $T = c / 4 f_o v_{max}$ where $v_{max}$ is the desired maximum micro-Doppler velocity resolution in the spectrum, c is the light speed and $f_o$ is the carrier frequency.

According possible embodiments of the invention, the resampling step comprises selecting, for each slot, the CIR value sampled at the time instant closest to the slot center and, if no sample is obtained in a slot, the CIR window slot sample is considered missing.

According to possible embodiments, the method further comprises a step of scheduling the additional CIR estimation fields to be transmitted in the current window according to a predefined scheduling policy. For example, such scheduling policy is to transmit K additional CIR estimation fields in the last K slots of the time window. A possible implementation of the scheduling policy provides that it is performed at half of the window duration W/2 and the subsequent window is shifted forward by W/2 slots.

According to possible embodiments, once the scheduling has been performed, for any CIR estimate extracted from a communication packet that is received after the scheduling operation, the first scheduled CIR estimation field is removed from the schedule.

According to possible embodiments, the method of the invention implements a reconstruction algorithm comprising:

a) building an W by W inverse Fourier basis matrix (B) wherein W is the number of slots in the time window;
b) building a reduced inverse Fourier matrix (F) containing the rows of B with indices corresponding to the non-missing CIR samples in the window;
c) posing an optimization problem such that its solution, a vector H of dimension W, is the Fourier transform of the complete CIR measurement window, and such that it enforces the sparsity of H;
d) solving said optimization problem to obtain H and computing the spectrum as $H^2$.

Preferably, the reconstruction can be performed only on the path yielding the highest received power or, in alternative, on a subset of the paths contained in the estimated CIR, being the subset containing the contribution of a target of interest.

In the following, a more detailed description of the features described above will be given with the support of related mathematics concept and principles the following description refers to possible combinations of the steps step of the method as above described, as a single embodiment. This is done in order to render the description as clear and fluent as possible. However, it is to be intended that different embodiments could be provided, using different combinations of features.

*CIR resampling*

According to the invention the CIR are sampled at time instants $t_i$, which coincide with the reception of the reflections from the $i$-th transmitted packet. To reconstruct the $\mu$D spectrum from CIR samples which are randomly distributed in the time domain, it is firstly resampled the CIR to obtain regularly spaced samples with a fixed granularity $T_c$ where possible.

To do so, it is resorted to the slotted resampling technique, which allows approximating a sequence of randomly spaced samples into a regular grid with missing values. $N_s$ consecutive samples are considered, obtained at the time instants $t_0, t_1, ..., t_{N_s}$ and denote by $0, T_c, 2T_c, ..., (K-1)T_c$ the regular grid with step size $Tc$. Slotted resampling constructs a new CIR sample sequence $h_{\ell,b}(kT_c)$ where the CIR values are obtained from the original sequence $h_{\ell,b}(t_i)$ as follows. Time bins (or intervals) of length $T_c$ are centered on each time instant of the regular grid, i.e., bin $k$ is $\beta_k = [kT_c - T_c/2, kT_c + T_c/2)$, with center $kT_c$. Then, the value of the CIR corresponding to the grid value $k$ is either (i) selected among the values of the original sequence whose sampling times fall inside bin $k$, taking the one whose sampling time is the closest to the bin center, or (ii) considered as a missing value if no samples of the original sequence fall inside bin $k$. Specifically,

$$h_{\ell,b}(kT_c) = \begin{cases} 0 & \text{if } \{t_i|t_i \in \beta_k\} = \emptyset, \\ h_{\ell,b}(t_k) & \text{otherwise,} \end{cases} \qquad (2)$$

where the 0 values represent missing samples and

$$t_k = \arg\min_{\tau \in \{t_i|t_i \in \beta_k\}} |kT_c - \tau|. \qquad (3)$$

The resulting, regularly spaced sequence of CIR samples is used to reconstruct the $\mu$D spectrum of the subject. However, due to the missing samples which are set to 0, a plain application of the STFT (as described above) would lead to a corrupted spectrum.

In the following the solution to this problem is detailed, which is based on sparse recovery techniques.

*Sparse $\mu$D recovery problem formulation*

Several methods exist to tackle the problem of computing the power spectrum of non-uniformly sampled signals. Such approach belongs to the category of sparsity-based approaches, in which the sparsity of the signal in the frequency domain is leveraged to drastically reduce the number of measurements needed for an accurate reconstruction of the spectrum. Windows of length $W$ samples (window size) every $\delta$ samples (overlap) from the sequence $h_{\ell,b}(kT_c)$ are selected, choosing $\delta = W/2$. Due to the slotted resampling process, each window may contain missing samples. The set of indices of the available samples contained in the m-th window is denoted by $\mathcal{U}_m$.

Then, it is defined vector $\mathbf{h}_{\ell,b}(m) \in \mathbb{C}^{|\mathcal{U}_m|}$, containing the available CIR samples in the m-th window, and vector $\widetilde{\mathbf{h}}_{\ell,b}(m) \in \mathbb{C}^W$, representing the complete $m$-th CIR window, which is only partially known due to the missing samples. It is also denoted by $\mathbf{F}_{inv}$ the inverse Fourier matrix, whose element in position ($g$, $l$) is given by

$$\left(\mathbf{F}_{inv}\right)_{gl} = (1/\sqrt{W})\exp(j2\pi gl/W)$$ , $g$, $l = 0, ..., W$ - 1 while $\mathbf{U}_m = \left[\mathbf{u}_i^T\right]$, $\forall i \in \mathcal{U}_m$, $\forall i \in$ Um is the matrix that selects the rows of $\mathbf{F}_{inv}$ whose indices are in $\mathcal{U}_m$. $\mathbf{u}_i$ is the vector of all zeros but the *i*-th component, which equals 1.

The following relation holds between the incomplete CIR window, $\mathbf{h}_{\ell,b}(m)$, and the Fourier Transform (FT) of the full CIR window, $\mathbf{H}_{\ell,b}(m) \in \mathbb{C}^W$ , which it is aimed to recover in order to compute the $\mu$D spectrum,

$$\mathbf{h}_{\ell,b}(m) = \mathbf{U}_m \tilde{\mathbf{h}}_{\ell,b}(m) = \mathbf{U}_m \mathbf{F}_{inv} \mathbf{H}_{\ell,b}(m) = \mathbf{\Psi}_m \mathbf{H}_{\ell,b}(m), \qquad (4)$$

wherein the last step matrix $\mathbf{\Psi}_m = \mathbf{U}_m \mathbf{F}_{inv}$ is used as a shorthand notation. Given Eq. (4), the aim is to recover $\mathbf{H}_{\ell,b}(m)$ from the incomplete measurement vector $\mathbf{h}_{\ell,b}(m)$, which is a typical sparse recovery or compressed sensing problem. In this framework, it has been proven that recovering the FT of the desired signal is possible if the latter is sparse in the frequency domain, i.e., the FT only contains a low fraction of non-zero elements. To verify that this sparsity assumption holds in this case, Eq. (1) can be rewritten after the resampling and windowing operations, so that the i-th sample of the complete m-th window is given by

$$\left[\tilde{\mathbf{h}}_{\ell,b}(m)\right]_i = \sum_{p=1}^{P_\ell(m)} a_{\ell,b}^p(m)\exp\left\{-j4\pi\frac{f_0}{c}\left[d_\ell^p + (m\delta + i)T_c v_{\ell,m}^p\right]\right\}, \qquad (5)$$

where $v_{\ell,m}^p$ is the radial velocity of the *p*-th reflector in path $\ell$ during window $m$, and $d_\ell^p$ its distance from the AP. Here, it is assumed that the velocity of each reflector can be considered constant during a window. In addition, it is also considered that the reflective coefficients and the number of reflectors are constant. This is reasonable for the considered setup, where the reflectors are parts of the human body, which typically move slowly compared to the duration of a window $WT_c$.

### Single-path sparse recovery

Given the model from Eq. (4), the reconstruction of the CIR FT along each path can be posed as a sparse recovery problem. Specifically, it is sought a vector $\mathbf{H}_{\ell,b^*}(m)$ which is a solution to Eq. (4) while being as sparse as possible, coherently with the above discussion.

Considering the BP $b^*$ pointing in the direction of the target, the desired FT of $\tilde{\mathbf{h}}_{\ell,b^*}(m)$ can be found as a solution of the optimization problem

$$\mathbf{H}_{\ell,b^*}(m) = \arg\min_{\mathbf{H}}||\mathbf{H}||_0 \quad \text{s. t. } ||\mathbf{h}_{\ell,b^*}(m) - \mathbf{\Psi}_m \mathbf{H}||_2 \leq \varepsilon, \qquad (6)$$

where $\|\cdot\|_0$ denotes the $\ell_0$-norm of a vector, i.e., the number of its non-zero components. The constant $\varepsilon > 0$ can be estimated from the noise in the CIR, using a training dataset.

An approximate local solution to Eq. (6) can be found using fast greedy algorithms. It is adopted the IHT, which solves

$$\mathbf{H}_{\ell,b^*}(m) = \arg\min_{\mathbf{H}}||\mathbf{h}_{\ell,b^*}(m) - \mathbf{\Psi}_m \mathbf{H}||_2^2 \quad \text{s. t. } ||\mathbf{H}||_0 \leq \Omega, \qquad (7)$$

where $\Omega$ is a pre-defined sparsity level parameter. The algorithm involves an iterative gradient descent step on the quadratic term in Eq. (7), followed by a thresholding operation:

$$\hat{\mathbf{H}}^{(n+1)} \leftarrow \mathcal{T}_\Omega\left[\hat{\mathbf{H}}^{(n)} + \eta\mathbf{\Psi}_m^T\left(\mathbf{h}_{\ell,b^*}(m) - \mathbf{\Psi}_m \hat{\mathbf{H}}^{(n)}\right)\right], \qquad (8)$$

where *n* is the iteration index and T$\Omega$ is the hard-thresholding operator, which sets to 0 all the components of the argument vector except the $\Omega$ largest ones in terms of the Euclidean norm. $\eta$ is a learning rate parameter which can be tuned to improve the convergence properties. The iterative process is stopped whenever $\|\hat{\mathbf{H}}^{(n+1)} - \tilde{\mathbf{H}}^{(n)}\|_2 < \xi$ or when a maximum number of iterations, $n$max, is reached. According to the present invention, $\Omega$ is a key parameter, which is strictly related to the number of reflectors $P_\ell(m)$: indeed, as IHT reconstructs a vector which has at most $\Omega$ non-zero elements, $\Omega$ is an upper bound for $P_\ell(m)$, and it can be thought of as the maximum number of reflectors per path that are allowed to be reconstructed. $\Omega$ can be tuned in order to obtain better $\mu$D reconstruction. The sparse recovery algorithm is summarized

in **Algorithm 1.**

---

**Algorithm 1** - Single path sparse recovery.

---

**Input:** $\mathbf{h}_{\ell,b^*}(m)$, $\eta$, $n_{max}$, $\Omega$, $\xi$.

**Output:** $\mathbf{H}_{\ell,b^*}(m)$.

1: Collect the set of available samples indices $\mathcal{U}_m$.

2: Build matrices $\mathbf{U}_m = \left[\mathbf{u}_i^T\right], \forall i \in \mathcal{U}_m$ and $\mathcal{F}_{inv}$

3: Compute $\mathbf{\Psi}_m = \mathbf{U}_m \mathcal{F}_{inv}$.

4: Set $\widehat{\mathbf{H}}^{(0)} = \mathbf{0}$, $n = 0$, $\gamma^{(0)}$ to any value $> \xi$.

5: **while** $n < n$ max or $\gamma(n) > \xi$ **do**

    6:    $\widehat{\mathbf{H}}^{(n+1)} \leftarrow$ Eq. (8)

    7:    $\gamma^{(n+1)} \leftarrow \|\widehat{\mathbf{H}}^{(n+1)} - \widehat{\mathbf{H}}^{(n)}\|_2$

    8:    $n \leftarrow n + 1$

9: **end while**

10: **return** $\widehat{\mathbf{H}}^{(n)}$

---

According to the compressive sensing theory, the reconstruction performance of IHT (and in general of any recovery algorithm) degrades as the number of available measurements, $|\mathcal{U}_m|$, decreases. Theoretical results show that the minimum number of measurements needed to reconstruct $\mathbf{H}_{\ell,b^*}(m)$ is $\mathcal{O}(\Omega\log(W/\Omega))$, although the exact number needs to be estimated empirically as it also depends on the level of noise present in the signal. In the following it will be demonstrated that the present invention can achieve excellent $\mu$D reconstruction with as low as W/8 measurements per window, thanks to the high sparsity of the mmWave CIR.

*Multi-path aggregation*

The moving body of a person causes several reflections that affect more than one CIR path, as already discussed. Using the described procedure, the present invention is able to retrieve the contribution of each path $\mathbf{H}_{\ell,b^*}(m)$ to the $\mu$D. Since the different body parts contribute to the $\mu$D in different paths, to fully capture human movement it is needd to combine the information from the different paths. It is called $Q$ the number of paths considered in obtaining the $\mu$D spectrum. The spectra obtained by the path caused by the torso, $\ell^*$, are aggregated with the $\lfloor Q/2 \rfloor$ paths preceding $\ell^*$ and the $\lfloor Q/2 \rfloor$ subsequent paths, as they may contain the contributions of the other body parts. The expression of the total $\mu$D spectrum is

$$\mathbf{D}(m) = \sum_{\ell=\ell^*-\lfloor Q/2 \rfloor}^{\ell^*+\lfloor Q/2 \rfloor} \left|\mathbf{H}_{\ell,b^*}(m)\right|^2, \qquad (9)$$

where the squared magnitude is applied element-wise. In addition, normalization of the spectra in the range [0, 1] is applied by computing $\mathbf{D}(m) \leftarrow \dfrac{\mathbf{D}(m) - \min_i \mathbf{D}_i(m)}{\max_i \mathbf{D}_i(m) - \min_i \mathbf{D}_i(m)}$.

Note that Eq. (9) entails solving $Q$ optimization problems of the form in Eq. (7), however, the $Q$ problems can be parallelized as they are completely independent. Decomposing the full $\mu$D spectrum reconstruction problem into $Q$ subproblems effectively allows applying sparse recovery techniques, which in turn lead to a significant reduction of the number of measurements needed.

The value of $Q$ is selected here due to physical considerations and validated in practice. The $\mu$D vectors from Eq. (9) can be collected in sequences, one every $\delta$ slots, forming $\mu$D spectrograms of arbitrary length, depending on the specific application that is being performed, e.g., activity recognition, fall detection, gait segmentation, etc. In the following, reference will be made to the number of $\mu$D vectors considered in such spectrograms as $\Lambda$.

*Sensing unit injection*

The present invention can exploit the sensing units in sparsely distributed communication packets to recover the $\mu$D spectrum of human movement.

However, during communication between the AP and one or more terminals it may happen that the AP remains silent for longer than the duration of a processing window, $W$, or that the number of received packets is less than the minimum number of measurements required for an accurate $\mu$D reconstruction. In these cases, the sparse recovery algorithm cannot recover $\mathbf{H}_{\ell,b}(m)$ as the available sensing units are insufficient. To tackle this problem, the system is allowed to inject sensing units into the channel whenever the number of communication packets is not sufficient for Algorithm 1 to work, i.e., when less than two CIR measurements are available in a window. Different from existing ISAC frameworks, however, the used sparse recovery approach allows to introduce a minimal amount of overhead, as the $\mu$D spectrum can be recovered from a number of CIR samples which is much lower than the full length of the window $W$. Note that for the injection of a sensing unit it is sufficient to transmit the necessary CIR estimation fields, without any preamble and header as used in conventional packets, since the unit is only received at the AP itself and contains a known waveform.

*Basis of the injection algorithm*

In the following, the proposed injection procedure will be presented as if both communication packets and sensing units were transmitted at times that lie on a uniform grid with spacing $T_c$. This simplification is based on the fact that the already described slotted resampling process is used. Therefore, the injection process can be described in terms of windows of size $W$, where each value in the window occupies a slot which is a multiple of $T_c$. Due to slotted resampling, the slots can be empty if no packet was transmitted sufficiently close to it.

The approach consists in setting a minimum number of sensing units per window, termed, that allows a sufficiently accurate reconstruction of the $\mu$D signatures.

Then additional units are transmitted whenever the number of reflections of communication packets in the window is not sufficient to meet this minimum requirement.

The proposed method only requires the knowledge of whether a reflected communication packet is received in the current slot, i.e., no information about the future traffic pattern is needed.

---

**Algorithm 2** - Injection of sensing units in window $m$.

---

**Input:** $M_s$.

1: *# P1 - observation phase*

2: $N(m) \leftarrow$ no. of sensing units received in the first half of the window

(either from reflected communications packets or injected).

3: *# P2 - scheduling phase*

4: $N_w(m) \leftarrow \max(M_s - N_a(m), 0)$.

5: Schedule $S_m = \{s_1, \ldots, s_{Nw}(m)\}$.

6: *# P3 - transmission phase*

7: **for** $q = mW/2, \ldots, (m+1)W/2 - 1$ **do**

```
8:    if q ∈ Sₘ then
9:       if no reflected comm. packet received then
10:         Transmit the sensing unit.
11:            Sₘ ← Sₘ \ {q}.
12:       else
13:         Use the sensing unit from the comm. packet
14:            Sₘ ← Sₘ \ {q}.
15:       end if
16:    else
17:       if reflected comm. packet received then
18:         Use the sensing unit from the comm. packet
19:            Sₘ ← Sₘ \ {minₛ∈S }.
20:       end if
21:    end if
22: end for
```

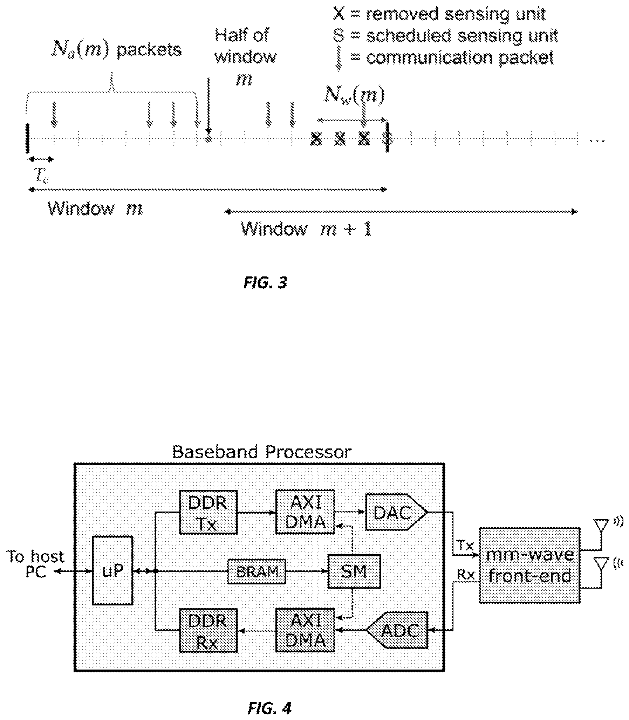

The algorithm, summarized in the above **Algorithm 2,** operates in three phases, namely *observation* (P1), *scheduling* (P2) and *transmission* (P3). Recall that the $\mu$D extraction already described follows a window-based approach, with subsequent windows overlapping by half of their length, as shown in Fig. 3.

Consider a time instant between the end of window $m-1$ and the start of window m + 1. This coincides with the half of window $m$, which is between slots $mW/2 - 1$ and $mW/2$. In this time instant it can be observed how many reflected communication packets were received in the first half of window $m$, which spans the indices from $(m-1)W/2$ to $mW/2-1$ (P1, line 2 in Algorithm 2). This number is denoted as $N_a(m)$. The whole injection algorithm operates on a half-window basis, as this allows reasoning on the sole current window $m$, as window $m-1$ has ended and window $m+1$ has not yet started. Based on $N_a(m)$, can computed how many sensing units would need in the remaining half of window m in order to meet the requirement of at least $M_s$ units, which is denoted by $N_w(m) = \max(M_s - N_a(m), 0)$. However, the sensing process has no knowledge of when future communication packets will be received, so the best that it can be done is to schedule the transmission of $(m)$ sensing units in the next half-window. The slots in which these packets are scheduled can be selected according to a deterministic rule or a probability distribution. It is called $S_m = \{s_1, \ldots, s_{N_w}(m)\}$ the set of indices of the slots in which the additional sensing units for the next half-window (P2, lines 4-5 in Algorithm 2) are scheduled. While P1 and P2 are performed in a single time slot, before the second half of window $m$ starts, P3 (lines 7-23 of Algorithm 2) is a dynamic process that spans the whole second half of window m. The indices of the slots considered in this part of the algorithm are $q = mW/2, \ldots, (m+1)W/2 - 1$. Note that some unknown communication packets may be received in this second half-window. The procedure iterates over the slots and in each of them checks if a sensing unit was scheduled for that slot, i.e., if $q \in S_m$. There are four possible cases:

(1) $q \in S_m$ and no communication packet was received in this slot. In this case the sensing unit is transmitted, $q$ removed from $S_m$.
(2) $q \in S_m$ and a communication packet (or more) was received in this slot. In this case the sensing unit is reused in the communication packet and $q$ removed from $S_m$.
(3) $q \notin S_m$ and no communication packet was received in this slot.
In this case just move to the next slot without taking action.
(4) $q \notin S_m$ and a communication packet (or more) was received in this slot. In this case the sensing unit is reused in the communication packet, then the next sensing unit is removed from the scheduled ones, i.e., $S_m$ is set: $S_m \leftarrow S_m \setminus \{\min_{s \in S_m} s\}$.

Note that, despite operating on a half-window basis, due to the overlap of adjacent windows, the algorithm only poses a constraint on the minimum number of packets sent per full window. This means that half a window can be empty as long as enough sensing units are received in the other half.

*Scheduling the sensing units.*

While P2, the scheduling of the sensing units, can be done with any arbitrary policy that guarantees that exactly $N_w(m)$ packets are scheduled in the next half window, the aim is to maximize the number of sensing units that can be piggybacked on communication packets, rather than using a dedicated transmission. From P3 in Algorithm 2, one can see that scheduling the sensing units towards the end of the half-window leaves more time for possible communication packets to become available and thus be reused instead of injecting a new sensing unit. Consequently, according to the present invention, the sensing units for the second half of window $m$ as a burst of packets spaced by $T_c$ are scheduled, which occupy the last $(m)$ slots of the window.

*A possible exemplary embodiment of the computer implemented method*

In the following, a possible embodiment of the computer implemented method of the invention is proposed, in view of the above description.

Step1.

Select a slot duration $T$ according to the desired maximum Doppler velocity of interest using the formula $T = c / 4 f_o v_{max}$ where $v_{max}$ is the desired maximum micro-Doppler velocity resolution in the spectrum, c is the light speed and $f_o$ is the carrier frequency.

Step2.

Construct a sliding time window of length W slots. At each time frame, shift the window forward by an arbitrary frame duration, e.g., half of the window length (W/2).

Step3.

The transmitter is used to transmit communication packets according to an underlying and ongoing communication process. The communication packets may contain CIR estimation fields, used by the processor to estimate the CIR. The communication can be performed using any communication protocol that supports, natively or via super resolution algorithms, a decimeter-level resolution in estimating the multiple paths in the CIR. These include mmWave communication systems such as IEEE 802.11ad/ay WLANs, 5G-NR (that naturally achieve high multipath estimation resolution) and sub 6GHz communication standards such as IEEE 802.11a/g/n/ac (that can use super-resolution to achieve high multipath resolution).

S4. Set an arbitrary integer threshold number of CIR samples needed in each window to obtain a good reconstruction of the CIR. The threshold can be set empirically or from theoretical bounds from sparse reconstruction theory.

Step 5.

At a pre-defined moment inside the window, if less than the threshold number of CIR estimates could be obtained from communication packets only, the processor schedules additional CIR estimation packets (containing only the CIR estimation field) to be transmitted in the current window, following an arbitrary policy, e.g., to transmit the K needed fields in the last K slots of the current window.

Step 6.

During the period of the current window that comes after the scheduling moment, the transmitter monitors how many communication packets containing CIR estimation fields are transmitted. For each such packet, the first additional remaining scheduled CIR estimation field is removed from the schedule as it is no longer needed to meet the threshold number of CIR measurements.

Step 7.

The transmitter transmits the scheduled CIR estimation fields. The receiver receives these additional waveforms in addition to the communication packets and the processor uses them to obtain additional CIR samples.

Step 8.

The processor the CIR estimates, h(t), computed from CIR estimation fields received in the current window (either from communication packets or from additionally injected CIR estimation fields). Denote the number of such estimates as Ns, and each estimate is obtained at a sampling instant $t_i$ with i = 1, ..., Ns. The CIR estimates are time-varying complex vectors of dimension L, where L is the number of different propagation paths in the signal.

Step 9.

The processor resamples the Ns CIR estimates collected in the current window using the slotted resampling method. Starting from the available sequence h($t_i$) with i = 1, ..., Ns, operate as follows:

    1. Consider the current window as a regular grid with step size T and W total steps.
    2. Construct a set of bins, each identified by index k, defined as the intervals [kT - T/2, kT + T/2) with center kT.
    3. Obtain the resampled sequence h(kT) using the following rule. For each bin k:

        a. If no sampling instant ti falls inside bin k, h(kT) is considered as a missing sample;
        b. If at least one instant ti falls inside bin k, set h(kT) = h($t_k$) where $t_k$ is the instant closest to the bin center kT among all sampling instants falling inside bin k.

    4. Denote by U the number of available (non-missing) samples in the current window.

Step 10.

The processor constructs an U by W partial inverse Fourier matrix, F, as follows:

    1. Build the W by W complete inverse Fourier matrix, whose element nm is equal to $W^{-1/2}\exp\left(\frac{j2\pi nm}{W}\right)$, where j is the imaginary unit.
    2. Select, from the complete matrix, a U by W submatrix selecting the rows corresponding to the indices of the non-missing samples in the current window.

Step 11.

Select a subset of the paths where a target of interest is located, denoted by l1, l2, ..., lQ identified by index q. This subset can be obtained using any side information such as a target tracking method.

Step 12.

Consider now independently each propagation path, identified by index by l1, l2, ..., lQ. Denote by hq the U-dimensional complex vector of samples of the q-th selected path in the current window. Set up the following optimization problem

$$argmin_{H_q}\left\|h_q - FH_q\right\|_2^2 \qquad such\ that \quad \left\|H_q\right\|_0 < S$$

where $H_q$ is the Fourier transform of the (unknown) complete CIR path q in the current window (of dimension W), $\|.\|_x$ is the x-norm of the argument vector and S is a parameter to be selected for the specific application.

Step 13.

Solve the optimization problem using any sparse optimization method, e.g., the iterative hard thresholding algorithm. Repeat S6-S7 for all paths l1, l2, ..., lQ.

Step 14.

Compute the elementwise square magnitude (spectrum) of $H_q$ for the selected paths of interest.

Step 15.

Sum the spectra for the selected paths of interest, obtaining a total spectrum, H, for the current window.

Step 16.

Move forward the time window and repeat steps Step 3 to Step 10.

Step 17.

Stack together the spectrum vectors obtained from M adjacent windows to obtain the W by M micro-Doppler spectrogram.

*A possible preferred embodiment implementation*

In general terms, a system for joint communication and reconstruction of the micro-Doppler time-frequency spectrum from sparse channel measurements according to the invention comprises:

a) a transmitter configured for transmitting wireless communication signals through a multipath channel, including channel estimation fields;
b) a receiver, configured for receiving a wireless signal which is the reflection or refraction of the transmitted signal; and
c) a processor, configured for implementing a method according to anyone of the possible embodiments described above.

Preferably, the transmitter and the receiver can be realized as a single transceiver sharing a same antenna array working in full-duplex mode.

According to possible embodiments, the estimated CIR is from a backscatter channel.

The transmitter can be equipped with an antenna array and phase shifters for directional beamforming.

Further, the CIR can be estimated for each of the different beampatterns used during the transmission.

In the following, it will be described one of the possible embodiments of the present invention implemented on a mmWave SDR platform. The implementation is based on the IEEE 802.11ay WiFi protocol, as it operates in the unlicensed 60 GHz band and supports CIR estimation for different BPs.

Testbed

The open-source mm-FLEX experimentation platform has been reproduced for a baseline design. It uses an FPGA-based baseband processor which can generate, capture and process (custom or standard compliant) frames with up to 1.76 GHz of bandwidth.

The baseband processor is connected to mmWave RF frontends with phased antenna arrays and supports various front-ends to operate in different frequency bands, e.g., at 28 GHz or 60 GHz. In the remainder of this description it will be used a 60 GHz RF front-end which simplifies experimentation as this is an unlicensed band, but it is noted that simply by changing the RF front-end, the present invention can operate in a different band, e.g., for 5G-NR compatibility.

In order to implement a system according to the present invention, the functionalities of the testbed have been augmented to enable full-duplex operation, not only in the baseband processor but also in the RF front-end. A block diagram of the system is shown in Fig. 4. In the baseband processor, the operation of the system is controlled by a state machine (SM) which triggers an AXI-DMA that reads the I/Q samples from the DDR-TX memory and feeds them to the DACs. The SM also triggers another AXI-DMA in the receiver datapath that saves the receives samples in the DDRRX memory, i.e., both datapaths are synchronized and no packet detection is required. To support the variable IFS extracted from real (or artificially generated) traces, a block RAM memory (BRAM) has been included in the FPGA logic that stores the IFS that will be used in the experiments. The SM reads these values sequentially, introducing a delay in the system according to the value read from memory. The variable IFS functionality can be disabled at runtime to configure a fixed IFS. It is remarked that since the up/down conversion stages are simultaneously used from the same mmWave development kit, the Tx/Rx subsystems are fed by the same local oscillator and thus the Carrier Frequency Offset (CFO) is very low (< 100 Hz), which enables the extraction of the $\mu$D values required by the present invention.

IEEE 802.11ay CIR estimation details. In IEEE 802.11ay, in-packet beam tracking is introduced, where the CIR is estimated using different BPs within a single packet. This is done by appending a given number of training (TRN) fields to the packet. A TRN field is composed of 6 TRN units formed by complementary Golay sequences of 128 BPSK modulated samples, for a total of 768 samples.

16

**Table 1: Summary of the implementation parameters.**

**The suggested values based on experimental results are shown in bold.**

| System parameters | | |
|---|---|---|
| Grid step | $T_c$ | 0.27 ms |
| Window length | $W$ | 64 |
| Window overlap | $\delta$ | 32 |
| Sparsity parameter | $\Omega$ | {1, 2, **3**, 4, 5, 6, 7} |
| No. aggregated paths | $Q$ | {1, 3, 5, 7, **9,** 11, 13, 15} |
| Min. no. measurements | $Ms$ | {4, **8**, 16, 24, 32, 64} |
| IHT learning rate | $\eta$ | 1 |
| IHT convergence threshold | $\xi$ | $10^{-4}$ |
| IHT maximum iteration number | $n_{max}$ | 200 |

According to the invention $n_{TRN}$ TRN fields are used as the sensing unit, where each TRN field employs a different BP, and $n_{TRN}$ is the number of subjects tracked by the system, as it suffices to use 1 TRN field per subject. Considering the typical number of people that are simultaneously tracked in human sensing systems, reasonable $n_{TRN}$ values go from 1 to 10. The CIR estimates obtained from each TRN field (BP) are then used as the inputs.

In Table 1 the system parameters used in the implementation are summarized. It is set $T_c$ = 0.27 ms and $W$ = 64, which lead to (i) a velocity resolution of $\Delta v = \frac{c}{2 f_o W T_c} \approx 0.14 \text{ ms}$ and (ii) aliasing-free velocity measurements up to of

$$v_{max} = \pm \frac{c}{4 f_o T_c} \approx \pm 4.48 \text{ m/s}$$ . These values are not critical to the functioning of the system, and can be modified according to specific implementation requirements. However, for reliable $\mu$D extraction without aliasing, it is advisable to adjust $Tc$ to a value that allows capturing the range of velocities typically covered by human movement, e.g., approximately $\pm(2 \text{ to } 3)$ m/s for a walking person, and up to $\pm 5$ m/s for running or other fast movements. Note that suitable values of $T_c$ can also be obtained in 5G-NR systems, where a base station can transmit downlink CSI-RS frames with a periodicity between 0.3125 ms and 80 ms. For a 5G-NR carrier frequency of 28 GHz, using $T_c$ = 0.3125 ms leads to $v_{max} \approx \pm 8.57$ m/s, which is enough to capture fast human movement.

For people tracking, periodically transmitted in-packet beam training frames are used with twelve TRN units and antenna beams covering a FOV range from -45° to 45°. Then, the already described distance and AoA estimation procedure are applied. Different values of $Ms$, $\Omega$ and $Q$ are experimented, as reported in Table 1 and already described, while for the IHT algorithm the parameters that led to the most accurate convergence results on the experiments are selected, i.e., $\eta$ = 1, $\xi$ = 10-4 and $n_{max}$ = 200.

In the following, the experimental results obtained with the above embodiment will be presented. The experiments were performed in a laboratory of 6×7 meters with a complex multi-path environment due to additional reflections caused by furniture, computers, screens, and a wide whiteboard.

*Results on synthetic traces*

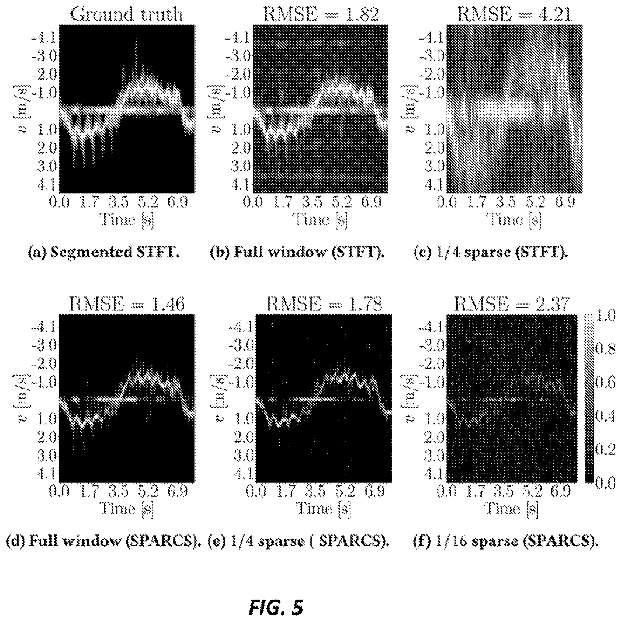

As a first qualitative result, the $\mu$D spectrograms obtained by the present invention on randomly sampled CIRs of a walking subject are shown in Figure 5. For this, synthetic traces are used, generated by measuring the CIR using a uniform sampling interval equal to $T_c$, and then setting to 0 a variable number of uniformly distributed values per window to simulate missing samples. This is a simplified case, as (i) the available (not removed) packets lie on a regular grid with spacing $T_c$, therefore no approximation error is introduced by slotted resampling, and (ii) samples are removed on a per window basis, so a minimum number of packets in each window is guaranteed. Still, this evaluation is useful to highlight the impact of increasing the sparsity level of the measurements for the present invention compared to standard STFT. In the results here presented, no packet injection is performed, as the aim is to assess the impact of the number of measurements per window on the reconstructed $\mu$D. In Fig. 5b the baseline walking spectrogram obtained using the standard STFT using the full window of 64 samples is shown.

The spectrogram shows a typical walking $\mu$D modulation, with the contribution of the static clutter (the strong component at 0 velocity), of the torso (the strong oscillating component around $\pm 1.5$ m/s and the limbs (the faint contributions around the torso component).

Moreover, a certain amount of noise and interference is present, as shown by the non-zero background level and the horizontal lines at around $\pm 2$ m/s and $\pm 3.7$ m/s. In Fig. 5c, the same method is applied to windows with only 16 out of 64 the samples retained, while the rest is set to 0. The impact is very strong as it completely corrupts the useful structure in the

$\mu$D signature. From Fig. 5d to Fig. 5f the results obtained by the present invention are shown, on the same sequence, with 64, 16 and 4 samples out of 64, respectively. Above each figure, it is also report the Root Mean-Squared Error (RMSE) of the $\mu$D with respect to a ground truth spectrogram, shown in Fig. 5a. This was obtained from the STFT output with full measurement windows (Fig. 5b), by manually segmenting the useful $\mu$D spectrum containing the gait information and setting to 0 all the background noise and the interference lines. Two interesting aspects are observed. On the one hand, the algorithm of the present invention can successfully recover the $\mu$D spectrum even when a large fraction of the samples is missing, and the quality of the result decreases gradually with the sparsity of the available measurements. On the other hand, the present invention almost completely eliminates noise and interference that are present with standard STFT, thanks to the effect of the sparsity constraint in Eq. (7), obtaining a lower RMSE than STFT even when for the latter all measurements in the window are available. This aspect is the main reason why the present invention not only reduces the overhead needed for human sensing, but also improves its accuracy.

*Realistic traces: the pdx/vwave dataset*

Next, the performance of the present invention on realistic WiFi AP traces have been evaluated. This poses an experimental challenge, because commercial devices implementing the IEEE 802.11ay standard are not yet available, and no public datasets containing real traffic traces for the PHY layer of mmWave WiFi (IEEE 802.11ay/ad) exist. For this reason, the pdx/vwave dataset is used, containing real traffic traces captured in different real environments from WiFi APs employing a legacy (sub 6GHz) WiFi protocol. Specifically, three, over one hour long, traces from this dataset are used, called psu cs, library and ug, respectively. Traces collected in different environments are selected to represent different kinds of traffic patterns (see Table 2 below).

**Table 2: Details of the 3 sequences of the pdx/vwave dataset.**

| Trace | Environment | No. frames | Duration |
|-------|-------------|------------|----------|
| psu cs | University CS dept. | 260326 | 1 : 00 h |
| library | Public library | 1300671 | 4 : 00 h |
| ug | Coffee shop | 895721 | 2 : 34 h |

The pdx/vwave dataset includes information about the transmission instants and packet sizes of all packets outgoing from the considered AP. Exploiting this information, measurements are performed transmitting packets according to these time patterns, using the BRAM in the FPGA to store the desired transmission instants. On top of the existing pdx/vwave communication patterns the injection algorithm (Algorithm 2) to send additional sensing units when needed is used.

Despite the fact that in the pdx/vwave dataset a legacy WiFi protocol is used, entailing much smaller packets sizes than IEEE 802.11ay, it is still reasonable to use it to obtain realistic packet transmission patterns. While in the pdx/vwave dataset the maximum physical layer PDU size is PPDUpdx = 1.5kB (without packet aggregation), in IEEE 802.11ay three main transmission modes are defined, namely High Throughput (HT), Directional Multi Gigabit (DMG) and Very High Throughput (VHT), with maximum physical layer PDU sizes, PPDUay, of 65kB, 262kB and 4692kB, respectively. With the increase in the packet sizes, the data rates of mmWave systems have increased accordingly, and in IEEE 802.11ay they will range from 0.3Gbps to several Gbps. As a numerical example, the traffic patterns in pdx/vwave with a typical bitrate of 4 Mbps would correspond to a bitrate of 0.7 Gbps in DMG IEEE 802.11ay when using a packet size of 262 kB instead of 1.5 kB. Note that traces with a larger number of packets and smaller PDU sizes (as will likely be the case in real deployments) will simply increase the sensing accuracy and further reduce the overhead.

*Human activity recognition results*

To evaluate the quality of the $\mu$D spectrograms extracted by the present invention, such spectrograms are used as the input to a HAR method. Specifically, a standard approach is followed, training a deep neural network on a dataset of $\Lambda \times W$ dimensional $\mu$D spectrograms, with $\Lambda = 200$ (equivalent to $\approx 1.76$ s), in order to classify the movement performed by the person during that time. In order to provide a comparison with other IEEE 802.11ay HAR methods based on regular CIR sampling, such as RAPID, the following four activities are considered: walking, running, sitting and waving hands. For HAR, a standard Convolutional Neural Network (CNN) architecture is used, composed of four inception modules performing $1\times1$, $3\times3$ and $5\times5$ convolutions.

The number of filters used is 8, 16, 32 and 64 for the four modules, respectively. The convolutional blocks are followed by a fully-connected layer with 64 neurons, to which Dropout is applied, and a final Softmax layer with four outputs. The exponential-linear unit activation function is used after each layer.

*Training data*

A training dataset has been collected involving six different subjects performing the four activities, for a total duration of about twelve minutes each. This leads to over 400 partially overlapping, 1.76 s long, $\mu$D sequences per activity, which has been augmented as described shortly. Note that the training data only includes uniformly sampled CIR traces with sampling period $Tc$ = 0.27 ms. The CNN training is done for 80 epochs, using a learning rate of 10-4, the Adam optimizer and the cross-entropy loss function. In order to enhance the robustness of the CNN, an ad-hoc data augmentation strategy is applied: some of the CIR samples in each window of the training dataset are randomly removed, and then the IHT algorithm according to the invention is applied to reconstruct the spectrograms. The process is repeated using a sparsity level of 1/8, 1/4 and 1/2, enlarging the training dataset to four times its original size, for a total of approximately 1600 $\mu$D spectrograms per activity. A randomly selected subset of the training data (around 10%) was used as a validation set to tune the hyperparameters of the CNN.

*Test data*

The CNN has been tested on the $\mu$D spectrograms obtained from CIR samples collected using the pdx/vwave packet traces previously described. Four, randomly selected, 20s long traces (one per activity) are collected for each of the three sequences types (psu cs, library and ug). The experiments are repeated for different values of the minimum number of sensing units per window, $M_s$ = 4, 8, 16, 32, 64, for a total of sixty test sequences. The test data involves a single subject, which was not included in the training set.

HAR F1 score.

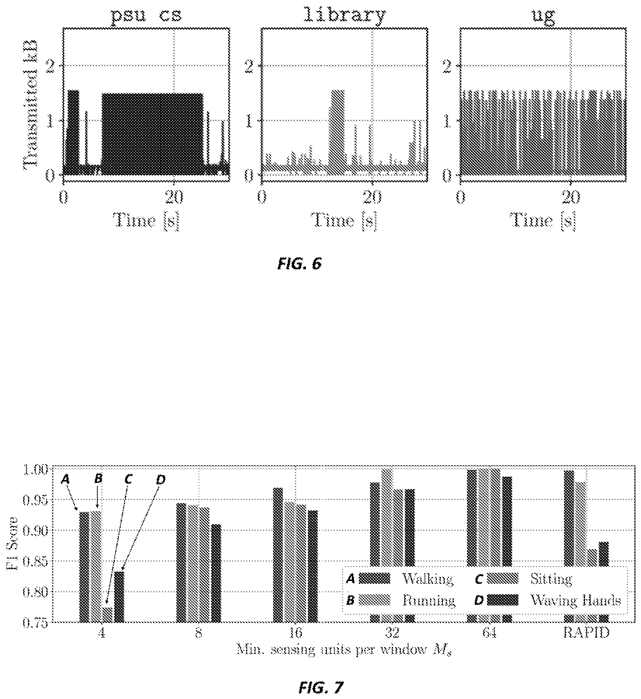

The performance of the CNN is evaluated with the per-class F1 score metric, which effectively summarizes the precision and recall and preserves the class-specific results. Fig. 7 shows the total average per-class F1 score over the 60 sequences, for different values of the minimum number of sensing units per window, $M_s$. As a baseline for comparison, the F1 score obtained by the RAPID algorithm it is also reported, which extracts the $\mu$D signatures by regularly sampling the CIR. The results show that the present invention can reach over 0.9 F1 scores on all activities with $M_s$ = 8 already, which corresponds to only 1/8 of the full measurements window. Notably, with $M_s$ = 4, the low number of measurements per window affects significantly only the 'Sitting' and 'Waving hands' activities, which involve fine-grained movements and are therefore more difficult to classify. Finally, the results from the present invention and RAPID are compared. For a fair comparison, RAPID's STFT has been implemented to extract the $\mu$D and trained the CNN on the resulting spectrograms without enlarging the dataset using different levels of sparsity described in the previous section. Instead, the training procedure of is directly used, since it has been found that the sparsity-based data augmentation slightly reduced RAPID's performance. It can be seen that sparse recovery problem formulation and enforcing a sparsity constraint on the individual paths is beneficial to HAR performance. The gap is particularly significant for 'Sitting' and 'Waving hands' as they involve lower energy traces in the spectrograms; these are more easily corrupted by noise and interference, that the present invention is mostly able to reject (see, again, the comparison between Fig. 5b and Fig. 5d).

*Overhead analysis*

Increasing $M_s$ to improve the HAR performance also increases the overhead of the present invention. A first general measure of this can be obtained comparing the maximum size of a PPDU in IEEE 802.11ay to the size of a sensing unit. Recalling the three different modes previously described and the size of an IEEE 802.11ay TRN field (768 bits), it is obtained that a sensing unit, with $n_{TRN}$ = 1, is 0.1%, 0.03% and 0.002% of a PPDU in HT, DMG and VHT, respectively. Moreover, the channel occupation time for a sensing unit with nTRN = 1 is 436ns, which is a negligible fraction (0.16%) of a slot of duration $T_c$.

Next, to evaluate the overhead of the present invention on a realistic communication scenario, the traces of the pdx/vwave dataset are used. In this way, it can also be assessed the impact of injecting sensing units, as they are not useful to the communication process. Denote by $ci$ the number of bits in the i-th communication packet transmitted in the trace. As the number of bits transmitted in each trace refers to a legacy, lower bitrate, WiFi protocol, the packet sizes according to the maximum PHY layer packet size in IEEE 802.11ay are rescaled. The size of packet $i$ in each trace as

$$\tilde{c}_i = \left( \frac{PPDUay}{PPDUpdx} \right) * c_i$$ are rescaled, with PPDUay = 262 kB. It is called $n_c$ the number of transmitted communication packets in a trace, by TRN$_{len}$ the length, in bits, of a piggybacked or injected TRN field, by $n_{inj}$ the number of injected sensing units and by nTRN the number of TRN fields used in every sensing operation (it is considered fixed, whereas in reality it is determined by the number of subjects in the environment). The overhead is defined as a function of $n_{inj}$ as

$$OH(n_{inj}) = \frac{n_{TRN}(n_C + n_{inj})\text{TRN}_{len}}{\sum_{i=1}^{n_C} \tilde{c}_i} \qquad (10)$$

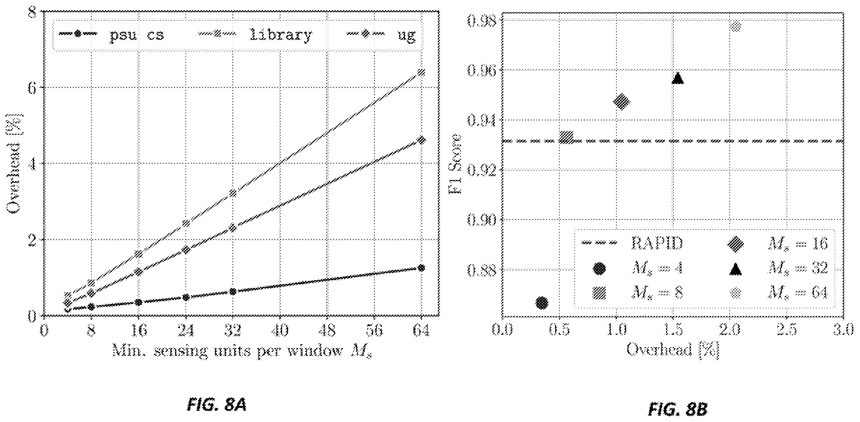

In Fig. 8A the overhead obtained on each of the three pdx/vwave traces is shown, using $n$TRN = 1. The overhead for different values of $n$TRN can be obtained by using it as a multiplicative factor on the values in Fig. 8A. It is noticed that the overhead scales almost linearly as $Ms$ is increased from 4 to 64. For values of $M_s < 32$, the entailed overhead is less than 4%, falling below 1% for $M_s = 8$ As a reference, the overhead for $M_s = 64$ is reported, which is the value obtained by injecting sensing units continuously into the channel, piggybacking them eventually on communication packets if possible. Note that existing approaches requiring uniform CIR sampling, like RAPID, would require an even higher overhead, as not only do they need 64 samples per window, but these samples have to be regularly spaced as no resampling procedure is carried out. This means they would have to take precedence over potential data packets so that they are sent exactly at the right sampling time.

From Fig. 8B, one can see that the present invention can achieve an F1 score of over 0.9 for every activity for a minimum of $M_s = 8$ sensing units per window, resulting in a sensing overhead of less than 1%. With this configuration, the present invention achieves a better F1 score than existing approaches, while reducing overhead by a factor of seven and being compatible with random access MAC protocols.

*Sensitivity to the choice of the parameters*

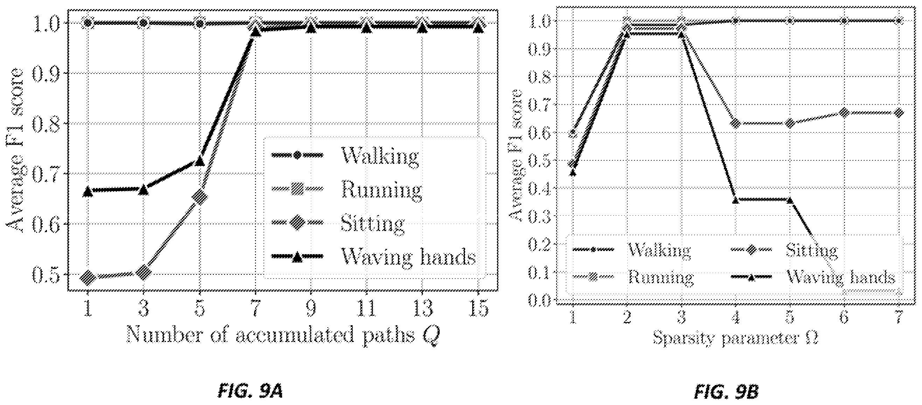

In Fig. 9, the effect of varying parameters $Q$ it is shown, representing the number of paths aggregated around the person's position (see Eq. (9)), and $\Omega$, which is the maximum number of resolvable Doppler components, equal to the sparsity parameter in the IHT algorithm.

The HAR per-class F1 score are computed using a random subset of the 60 test sequences. The values adopted in the experiments are reported in Table 1.

Impact of changing $Q$.

The results show that the present invention is robust to almost any value of $Q$ when considering walking and running, whereas sitting and waving hands are negatively affected by reducing $Q$ below seven. This is due to the fact that while walking and running are, in most cases, distinguishable even from the sole contribution of the torso, this is not true for sitting and waving hands that require including the reflection paths coming from the limbs. Computational complexity considerations are also in order for high values of $Q$, as it leads to solving $Q$ times Eq. (7) at each $\mu$D extraction process. As the problems are independent, they can be solved in parallel, and thus a reasonable approach is to tune $Q$ according to a trade-off between $\mu$D reconstruction accuracy and hardware resource availability for parallelization. In the following, $Q = 9$ is used. Considering that $B = 1.76$ GHz transmission bandwidth (one IEEE 802.11ay channel) is used, the range resolution is $c/2B = 8.5$ cm. This means that summing the contribution of $\lfloor Q/2 \rfloor$ paths before and after the one corresponding to the torso, a region of $\pm 34$ cm around the person's position is included in the spectrum, which is a reasonable value considering typical body sizes and that the subjects are moving.

Fixing $Q = 9$, in Fig. 9B, it is shown that the best values for $\Omega$ are 2 and 3 for all the activities.

This is because using $\Omega = 1$ often leads to only reconstructing the 0 Doppler component in the spectrogram, losing the information on the person's movement. On the other hand, choosing $\Omega$ too high makes the IHT reconstruction imprecise, as with a low number of measurements per window enforcing more sparsity is beneficial to restrict the number of possible solutions to Eq. (7).

In this description, the first ISAC system that can sense human $\mu$D signatures from irregular and sparse CIR estimates has been designed and implemented. These estimates are obtained in a standard compliant way by both reusing optional CIR estimation fields appended to communication packets and by the sporadic injection of sensing packets whenever communication traffic is absent. Different from the existing ISAC methods, the present invention is based on a sparse recovery approach to the $\mu$D reconstruction, which is theoretically grounded in the instrinsic sparse multi-path environment of the channel. This enables an accurate $\mu$D extraction from a significantly lower number of randomly distributed CIR samples, thus drastically reducing the sensing overhead. After a CIR resampling step along the time domain, the present invention performs an iterative sparse reconstruction in the frequency domain, decoupling different propagation paths at first, to leverage their sparsity property, and then combining them to obtain the final $\mu$D spectrum.

While the present invention is compatible with different communication systems (e.g., 3GPP 5G-NR, and IEEE WLANs), for the implementation an IEEE 802.11ay SDR platform working in the 60 GHz band has been used. The system has been tested on a large set of standard-compliant CIR traces matching the traffic patterns of real WiFi access points, performing a typical downstream application such as HAR. The results show that the present invention entails over seven times lower overhead compared to prior methods, while achieving better performance.

The present invention has been so far described with reference to preferred embodiments thereof. It is to be meant that each one of the technical solutions implemented in the preferred embodiments, herein described by way of example, can advantageously be combined, differently from what described, with the other ones, to create additional embodiments, the scope of the invention being defined by the appended claims.

**Claims**

1. A computer implemented method for joint communication and reconstruction of a micro-Doppler time-frequency spectrum from sparse channel measurements, wherein wireless communication signals, including channel estimation fields, are transmitted through a multi-path channel, and the reflections or refractions of the transmitted signal are received, the method comprising:

   i) estimating the channel impulse response (CIR) of the multi-path channel, wherein the CIR contains complex channel gains for each path of the multi-path channel, so as to obtain a plurality of CIR estimates corresponding to irregularly spaced CIR values;
   ii) resampling the available channel impulse response (CIR) estimates so as to obtain an incomplete regular grid using a resampling technique, the incomplete regular grid comprising CIR samples regularly spaced in time with possibly some missing samples;
   iii) performing a sparse reconstruction of a Fourier transform of the incomplete regular grid in the time domain for reconstructing the micro-Doppler time-frequency spectrum.

2. The method according to claim 1, wherein said resampling is performed on a slotted sliding window with slot duration (T) and window length slots (W), and wherein said steps i), ii) and iii) are repeated for each subsequent window.

3. The method according to claim 2, wherein said time slot duration (T) is selected as $T=c/(4f_o v_{max})$ where $v_{max}$ is the desired maximum micro-Doppler velocity resolution in the spectrum, c is the light speed and $f_o$ is the carrier frequency.

4. The method according to claim 2 or 3, wherein the following steps are performed between said estimating and resampling steps:

   b) setting a threshold number of CIR measurements-per-window, needed to reach the desired micro-Doppler reconstruction quality;
   c) transmitting a number of additional CIR estimation fields to meet the threshold number of measurements.

5. The method according to claim 4, further comprising a step of scheduling the additional CIR estimation fields to be transmitted in the current window according to a predefined scheduling policy.

6. The method according to claim 5, wherein said scheduling policy is to transmit K additional CIR estimation fields in the last K slots of the time window; wherein the scheduling is performed at half of the window duration (W/2) and the subsequent window is shifted forward by W/2 slots; and wherein, after the scheduling has been performed, for any CIR estimate extracted from a communication packet that is received after the scheduling operation, the first scheduled CIR estimation field is removed from the schedule.

7. The method according to one of the claims 2 to 6, wherein said resampling step comprises selecting, for each slot, the CIR value sampled at the time instant closest to the slot center and, if no sample is obtained in a slot, the CIR window slot sample is considered missing.

8. The method according to one of the claims 1 to 7, wherein said step of performing sparse reconstruction is performed separately for each signal propagation path.

9. The method according to one of the claims 2 to 8, implementing a reconstruction algorithm comprising:

   d) building an W by W inverse Fourier basis matrix (B) wherein W is the number of slots in the time window;
   e) building a reduced inverse Fourier matrix (F) containing the rows of B with indices corresponding to the non-missing CIR samples in the window;
   f) posing an optimization problem such that its solution, a vector H of dimension W, is the Fourier transform of the complete CIR measurement window, and such that it enforces the sparsity of H;

g) solving said optimization problem to obtain H and computing the spectrum as $H^2$.

10. The method according to claim 9, wherein the algorithm used for solving the optimization problem uses the Iterative Hard Thresholding (IHT) method.

11. The method according to one of the claims 1 to 10, wherein said reconstruction is performed only on the path yielding the highest received power.

12. The method according to one of the claims 1 to 10, wherein said reconstruction is performed on a subset of the paths contained in the estimated CIR, being the subset containing the contribution of a target of interest.

13. The method according to claim 12, wherein the spectra from the different paths of the subset are combined by summing the micro-Doppler spectra obtained from the Fourier transforms.

14. A system for joint communication and reconstruction of the micro-Doppler time-frequency spectrum from sparse channel measurements, comprising:

    h) a transmitter configured for transmitting wireless communication signals through a multipath channel, including channel estimation fields;
    i) a receiver, configured for receiving a wireless signal which is the reflection or refraction of the transmitted signal;
    j) a processor, configured for implementing a method according to anyone of the claims 1 to 13.

15. The system according to claim 14, wherein said transmitter and said receiver are a single transceiver sharing a same antenna array working in full-duplex mode.

16. The system according to claim 14 or 15, wherein the estimated CIR is from a backscatter channel.

17. The system according to one of the claims 14 to 16, wherein the transmitter is equipped with an antenna array and phase shifters for directional beamforming.

18. The system according to claim 17, wherein the CIR is estimated for each of the different beampatterns used during the transmission.

**Patentansprüche**

1. Computerimplementiertes Verfahren für eine gemeinsame Kommunikation und Rekonstruktion eines Mikro-Doppler-Zeit-Frequenz-Spektrums aus Sparse-Kanal-Messungen, wobei drahtlose Kommunikationssignale, einschließlich Kanalschätzungsfeldern, über einen Mehrwegekanal übertragen werden und die Reflexionen oder Brechungen des übertragenen Signals empfangen werden, das Verfahren umfassend:

    i) Schätzen der Kanalimpulsantwort (CIR) des Mehrwegekanals, wobei die CIR komplexe Kanalverstärkungen für jeden Pfad des Mehrwegekanals enthält, um eine Vielzahl von CIR-Schätzungen zu erhalten, die unregelmäßig beabstandeten CIR-Werten entsprechen;
    ii) Neuabtasten der verfügbaren Schätzungen der Kanalimpulsantwort (CIR), um unter Verwendung einer Neuabtastungstechnik ein unvollständiges regelmäßiges Raster zu erhalten, das unvollständige regelmäßige Raster umfassend zeitlich gleichmäßig verteilte CIR-Abtastwerte, wobei möglicherweise einige Abtastwerte fehlen;
    iii) Durchführen einer Sparse-Rekonstruktion einer Fourier-Transformation des unvollständigen regulären Gitters in dem Zeitbereich zum Rekonstruieren des Mikro-Doppler-Zeit-Frequenz-Spektrums.

2. Verfahren nach Anspruch 1, wobei das Neuabtasten an einem geschlitzten gleitenden Fenster mit Schlitzdauer (T) und Fensterlängenschlitzen (W) durchgeführt wird und wobei die Schritte i), ii) und iii) für jedes nachfolgende Fenster wiederholt werden.

3. Verfahren nach Anspruch 2, wobei die Zeitschlitzdauer (T) als $T=c/(4f_o v_{max})$ gewählt wird, wobei $v_{max}$ die gewünschte maximale Mikro-Doppler-Geschwindigkeitsauflösung in dem Spektrum ist, c die Lichtgeschwindigkeit ist und $f_o$ die

Trägerfrequenz ist.

4. Verfahren nach Anspruch 2 oder 3, wobei zwischen den Schätz- und Neuabtastschritten die folgenden Schritte durchgeführt werden:

b) Festlegen einer Schwellenanzahl von CIR-Messungen pro Fenster, die erforderlich sind, um die gewünschte Mikro-Doppler-Rekonstruktionsqualität zu erreichen;
c) Übertragen einer Anzahl von zusätzlichen CIR-Schätzfeldern, um die Schwellenanzahl an Messungen zu erreichen.

5. Verfahren nach Anspruch 4, ferner umfassend einen Schritt zum Planen der zusätzlichen CIR-Schätzfelder umfasst, die in dem aktuellen Fenster gemäß einer vordefinierten Planungsrichtlinie übertragen werden sollen.

6. Verfahren nach Anspruch 5, wobei die Planungsrichtlinie darin besteht, K zusätzliche CIR-Schätzfelder in den letzten K Schlitzen des Zeitfensters zu übertragen; wobei das Planen bei einer Hälfte der Fensterdauer (W/2) durchgeführt wird und das nachfolgende Fenster um W/2 Schlitze nach vorne verschoben wird; und wobei, nachdem das Planen durchgeführt wurde, für eine beliebige CIR-Schätzung, die aus einem Kommunikationspaket extrahiert wird, das nach der Planungsoperation empfangen wird, das erste geplante CIR-Schätzfeld aus der Planung entfernt wird.

7. Verfahren nach einem der Ansprüche 2 bis 6, wobei der Schritt des Neuabtastens das Auswählen, für jeden Schlitz, des CIR-Werts umfasst, der zu dem Zeitpunkt abgetastet wurde, der dem Schlitzmittelpunkt am nächsten liegt, und falls in einem Schlitz kein Abtastwert erhalten wird, der Abtastwert des CIR-Fensterschlitzes als fehlend betrachtet wird.

8. Verfahren nach einem der Ansprüche 1 bis 7, wobei der Schritt das Durchführen der Sparse-Rekonstruktion für jeden Signalausbreitungspfad separat durchgeführt wird.

9. Verfahren nach einem der Ansprüche 2 bis 8, das einen Rekonstruktionsalgorithmus implementiert, umfassend:

d) Erstellen einer W x W inversen Fourier-Basismatrix (B), wobei W die Anzahl der Schlitze in dem Zeitfenster ist;
e) Erstellen einer reduzierten inversen Fouriermatrix (F), die die Zeilen von B mit Indizes enthält, die den nicht fehlenden CIR-Abtastwerten in dem Fenster entsprechen;
f) derartiges Aufstellen eines Optimierungsproblems, das seine Lösung, ein Vektor H von Dimension W, die Fourier-Transformation des vollständigen CIR-Messfensters ist und derart, dass es die Spärlichkeit von H erzwingt;
g) Lösen des Optimierungsproblems, um H zu erhalten, und Berechnen des Spektrums als $H^2$.

10. Verfahren nach Anspruch 9, wobei der Algorithmus, der zum Lösen des Optimierungsproblems verwendet wird, das Iterative-Hard-Thresholding-Verfahren (IHT-Verfahren) verwendet.

11. Verfahren nach einem der Ansprüche 1 bis 10, wobei die Rekonstruktion nur auf dem Pfad durchgeführt wird, der die höchste Empfangsleistung liefert.

12. Verfahren nach einem der Ansprüche 1 bis 10, wobei die Rekonstruktion an einer Teilmenge der Pfade durchgeführt wird, die in der geschätzten CIR enthalten sind, wobei es sich um die Teilmenge handelt, die den Beitrag eines Ziels von Interesse enthält.

13. Verfahren nach Anspruch 12, wobei die Spektren aus den unterschiedlichen Pfaden der Teilmenge durch Summieren der Mikro-Doppler-Spektren kombiniert werden, die aus den Fourier-Transformationen erhalten werden.

14. System für die gemeinsame Kommunikation und Rekonstruktion des Mikro-Doppler-Zeit-Frequenz-Spektrums aus Sparse-Kanal-Messungen, umfassend:

h) einen Sender, der zum Übertragen drahtloser Kommunikationssignale über einen Mehrwegekanal konfiguriert ist, einschließlich Kanalschätzungsfeldern;
i) einen Empfänger, der zum Empfangen eines drahtlosen Signals konfiguriert ist, das die Reflexion oder Brechung des gesendeten Signals ist;
j) einen Prozessor, der zum Implementieren eines Verfahrens nach einem der Ansprüche 1 bis 13 konfiguriert ist.

15. System nach Anspruch 14, wobei der Sender und der Empfänger ein einzelner Sendeempfänger sind, der sich ein gemeinsames Antennenarray teilt, das in dem Vollduplexmodus arbeitet.

16. System nach Anspruch 14 oder 15, wobei der geschätzte CIR von einem Rückstreuungskanal stammt.

17. System nach einem der Ansprüche 14 bis 16, wobei der Sender mit einem Antennenarray und Phasenschiebern für eine gerichtete Strahlformung ausgestattet ist.

18. System nach Anspruch 17, wobei der CIR für jedes der unterschiedlichen Strahlmuster geschätzt wird, die während der Übertragung verwendet werden.

**Revendications**

1. Procédé implémenté par ordinateur destiné à une communication et une reconstruction conjointes d'un spectre temps-fréquence micro-Doppler à partir de mesures de canal clairsemées, dans lequel des signaux de communication sans fil, comportant des champs d'estimation de canal, sont transmis par le biais d'un canal à trajets multiples, et les réflexions ou réfractions du signal transmis sont reçues, le procédé comprenant :

   i) l'estimation de la réponse impulsionnelle de canal (CIR) du canal à trajets multiples, dans lequel la CIR contient des gains de canal complexes pour chaque trajet du canal à trajets multiples, de façon à obtenir une pluralité d'estimations de CIR correspondant à des valeurs de CIR irrégulièrement espacées ;
   ii) le rééchantillonnage des estimations de réponse impulsionnelle de canal (CIR) disponibles de façon à obtenir une grille régulière incomplète à l'aide d'une technique de rééchantillonnage, la grille régulière incomplète comprenant des échantillons de CIR espacés régulièrement dans le temps avec éventuellement quelques échantillons manquants ;
   iii) la mise en œuvre d'une reconstruction clairsemée d'une transformée de Fourier de la grille régulière incomplète dans le domaine temporel permettant de reconstruire le spectre temps-fréquence micro-Doppler.

2. Procédé selon la revendication 1, dans lequel ledit rééchantillonnage est mis en œuvre sur une fenêtre glissante à créneaux avec une durée de créneau (T) et des créneaux de longueur de fenêtre (W), et dans lequel lesdites étapes i), ii) et iii) sont répétées pour chaque fenêtre suivante.

3. Procédé selon la revendication 2, dans lequel ladite durée de créneau de temps (T) est sélectionnée en tant que $T=c/(4f_o v_{max})$ où $v_{max}$ est la résolution de vitesse micro-Doppler maximale souhaitée dans le spectre, c est la vitesse de la lumière et $f_o$ est la fréquence de porteuse.

4. Procédé selon la revendication 2 ou 3, dans lequel les étapes suivantes sont mises en œuvre entre lesdites étapes d'estimation et de rééchantillonnage :

   b) le réglage d'un nombre seuil de mesures de CIR par fenêtre, nécessaire pour atteindre la qualité souhaitée de reconstruction micro-Doppler ;
   c) la transmission d'un nombre de champs d'estimation de CIR supplémentaires pour respecter le nombre seuil de mesures.

5. Procédé selon la revendication 4, comprenant en outre une étape de planification des champs d'estimation de CIR supplémentaires à transmettre dans la fenêtre actuelle selon une politique de planification prédéfinie.

6. Procédé selon la revendication 5, dans lequel ladite politique de planification est de transmettre K champs d'estimation de CIR supplémentaires dans les K derniers créneaux de la fenêtre temporelle ; dans lequel la planification est mise en œuvre à la moitié de la durée de fenêtre (W/2) et la fenêtre suivante est décalée vers l'avant de W/2 créneaux ; et dans lequel, après que la planification a été mise en œuvre, pour l'une quelconque estimation de CIR extraite d'un paquet de communication qui est reçu après l'opération de planification, le premier champ d'estimation de CIR planifié est retiré de la planification.

7. Procédé selon l'une des revendications 2 à 6, dans lequel ladite étape de rééchantillonnage comprend la sélection, pour chaque créneau, de la valeur de CIR échantillonnée à l'instant le plus proche du centre de créneau et, si aucun échantillon n'est obtenu dans un créneau, l'échantillon de créneau de fenêtre de CIR est considéré comme manquant.

**8.** Procédé selon l'une des revendications 1 à 7, dans lequel ladite étape de mise en œuvre de reconstruction clairsemée est mise en œuvre séparément pour chaque trajet de propagation de signal.

**9.** Procédé selon l'une des revendications 2 à 8, implémentant un algorithme de reconstruction comprenant :

d) la construction d'une matrice de base de Fourier inverse W sur W (B) dans lequel W est le nombre de créneaux dans la fenêtre temporelle ;

e) la construction d'une matrice de Fourier inverse réduite (F) contenant les rangées de B avec des indices correspondant aux échantillons de CIR non manquants dans la fenêtre ;

f) le fait de poser un problème d'optimisation de telle sorte que sa solution, un vecteur H de dimension W, est la transformée de Fourier de la fenêtre de mesure de CIR complète, et de telle sorte qu'il impose le caractère clairsemé de H ;

g) la résolution dudit problème d'optimisation pour obtenir H et le calcul du spectre en tant que $H^2$.

**10.** Procédé selon la revendication 9, dans lequel l'algorithme utilisé pour la résolution du problème d'optimisation utilise le procédé de seuillage itératif dur (IHT).

**11.** Procédé selon l'une des revendications 1 à 10, dans lequel ladite reconstruction est mise en œuvre uniquement sur le trajet donnant la puissance reçue la plus élevée.

**12.** Procédé selon l'une des revendications 1 à 10, dans lequel ladite reconstruction est mise en œuvre sur un sous-ensemble des trajets contenus dans la CIR estimée, qui est le sous-ensemble contenant la contribution d'une cible d'intérêt.

**13.** Procédé selon la revendication 12, dans lequel les spectres provenant des différents trajets du sous-ensemble sont combinés par sommation des spectres micro-Doppler obtenus à partir des transformées de Fourier.

**14.** Système destiné à une communication et une reconstruction conjointes du spectre temps-fréquence micro-Doppler à partir de mesures de canal clairsemées, comprenant :

h) un transmetteur configuré pour la transmission de signaux de communication sans fil par le biais d'un canal à trajets multiples, comportant des champs d'estimation de canal ;

i) un récepteur, configuré pour recevoir un signal sans fil qui est la réflexion ou réfraction du signal transmis ;

j) un processeur, configuré pour l'implémentation d'un procédé selon l'une quelconque des revendications 1 à 13.

**15.** Système selon la revendication 14, dans lequel ledit transmetteur et ledit récepteur sont un unique transcepteur partageant un même réseau d'antennes travaillant en mode de duplex intégral.

**16.** Système selon la revendication 14 ou 15, dans lequel la CIR estimée provient d'un canal de rétrodiffusion.

**17.** Système selon l'une des revendications 14 à 16, dans lequel le transmetteur est équipé d'un réseau d'antennes et de déphaseurs pour une formation de faisceau directionnelle.

**18.** Système selon la revendication 17, dans lequel la CIR est estimée pour chacun des différents profils de faisceaux utilisés pendant la transmission.

**SPARCS**

Sparse communication traffic + injection

CIR estimation → CIR Resampling → Sensing units injection

People Tracking → Sparse Recovery

micro-Doppler

Radar-like uniform transmissions

CIR estimation → People Tracking → STFT → micro-Doppler

Traditional CIR-based sensing

**FIG. 1**

EP 4 518 751 B1

*FIG. 2*

*FIG. 3*

*FIG. 4*

(a) Segmented STFT.    (b) Full window (STFT).    (c) 1/4 sparse (STFT).

(d) Full window (SPARCS).   (e) 1/4 sparse ( SPARCS).    (f) 1/16 sparse (SPARCS).

**FIG. 5**

**FIG. 6**

**FIG. 7**

*FIG. 8B*

*FIG. 8A*

*FIG. 9B*

*FIG. 9A*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- RAPID: Retrofitting IEE 802.11ay Access Points for Indoor Human Detection and Sensing. **JACOPO PEGORARO et al.** ARXIV.ORG. 30 March 2022, 14853